(19) Europäisches Patentamt / European Patent Office / Office européen des brevets

(11) **EP 2 484 321 A1**

(12) **EUROPEAN PATENT APPLICATION**
published in accordance with Art. 153(4) EPC

(43) Date of publication:
**08.08.2012 Bulletin 2012/32**

(51) Int Cl.:
***A61F 13/49*** *(2006.01)*  ***A61F 13/53*** *(2006.01)*

(21) Application number: **10820296.1**

(22) Date of filing: **27.08.2010**

(86) International application number:
**PCT/JP2010/065083**

(87) International publication number:
**WO 2011/040176 (07.04.2011 Gazette 2011/14)**

(84) Designated Contracting States:
**AL AT BE BG CH CY CZ DE DK EE ES FI FR GB
GR HR HU IE IS IT LI LT LU LV MC MK MT NL NO
PL PT RO SE SI SK SM TR**

(30) Priority: **30.09.2009 JP 2009228370**

(71) Applicant: **Unicharm Corporation
Ehime 799-0111 (JP)**

(72) Inventors:
• **NOZAKI, Satoshi**
  **Kanonji-shi**
  **Kagawa 769-1602 (JP)**

• **SHIRAI, Tsutomu**
  **Kanonji-shi**
  **Kagawa 769-1602 (JP)**
• **TANIO, Toshiyuki**
  **Kanonji-shi**
  **Kagawa 769-1602 (JP)**

(74) Representative: **Peter, Julian
  Staeger & Sperling
  Partnerschaftsgesellschaft
  Sonnenstrasse 19
  80331 München (DE)**

(54) **ABSORBENT LAMINATED SHEET**

(57) It is an object of the invention to provide a layered absorbent sheet with excellent absorption properties including fluid retention, fluid diffusibility and absorbed fluid retention volume, and with particularly excellent deformation resistance against deformation by twisting and wrinkles.

A layered absorbent sheet comprising a first sheet layer on the absorbing side, a second sheet layer on the non-absorbing side, and a super-absorbent polymer layer between the first sheet layer and second sheet layer, wherein the first sheet and/or second sheet are bulky absorbent sheets, the bulky absorbent sheets comprising heat-expanding particles that are dispersed in the bulky absorbent sheet and have been expanded by heat.

Fig.13

(a)

(b)

(c)

**Description**

Technical Field

The present invention relates to a layered absorbent sheet to be used in an absorbent body of an absorbent article, such as a sanitary napkin or diaper. The invention further particularly relates to a layered absorbent sheet with low twisting and deformation during use, and with excellent fluid retention, fluid diffusibility and absorbed fluid retention volume.

Background Art

Absorbent articles, such as sanitary napkins and disposable diapers must be thin with high absorption capacity, and this trend is expected to further increase from the viewpoint of ecology, economy and product quality. To meet this demand, it has been attempted in the past to increase absorption using super-absorbent polymers in the absorbent body, while reducing the amount of other fiber materials to obtain thinner absorbent articles.

Absorbent body structures employing super-absorbent polymers have shifted from first-generation types to the current fourth-generation types. First-generation absorbent bodies have had a construction in which a polymer sheet is prepared comprising super-absorbent polymer particles integrally sandwiched by water-absorbing sheets, and is stacked with another material for absorbent bodies composed of a water-absorbing sheet, fluff pulp or the like. Second-generation absorbent bodies have had a construction with super-absorbent polymer particles directly dispersed in a fluff pulp layer. Third-generation absorbent bodies have had a construction with the amount of super-absorbent polymer particles increased over second-generation types, and with a reduced amount of fluff pulp.

The fourth-generation absorbent bodies that are currently being further developed for even greater economy of resources and smaller thicknesses, are targeted to have absorbent body structures composed mainly of super-absorbent polymer sheets and absorbent body structures composed only of super-absorbent polymer sheets, and much research is being conducted on super-absorbent polymers, their base sheets, and methods of anchoring super-absorbent polymers to base sheets.

However, as the proportion of super-absorbent polymer is increased and the absorbent body thickness is reduced, the absorbent body becomes more prone to twisting, and super-absorbent polymers that have swelled due to absorption of fluid produce a gel blocking phenomenon, blocking absorption and becoming prone to leaking. These drawbacks are particularly notable with absorbent body structures composed mainly of polymer sheets, which are the goal with fourth-generation types.

Much research has been conducted in order to overcome these drawbacks, and for example, PTL 1 discloses a method of dispersing a super-absorbent polymer on a wet paper sheet made using bulky cellulose fiber, such as cellulose fiber with a fiber roughness of at least 0.3 or with a cross-sectional deviation from roundness of 0.5-1, or crosslinked cellulose fiber, and then stacking a separate fiber aggregate thereover and drying the stack to integrate them, whereby there is obtained a thin absorbent sheet having the polymer dispersed in the interior, and exhibiting low shedding of the polymer and no gel blocking phenomenon.

PTL 2 discloses covering polymer particles that are dispersed on a nonwoven fabric base, with a combination of 2 hot-melt layers with different fiber thicknesses and mesh densities, to obtain a highly absorbent complex sheet with no shedding of the polymer and no inhibition of the swelling property.

PTL 3 discloses laminating a sheet layer composed of a fibrous base, such as a dry pulp nonwoven fabric, and a polymer layer comprising a super-absorbent polymer and a synthetic resin having a permanent deformation of no greater than 50% when subjected to 100% strain, and obtaining an integrated absorbent body, which has high shape retention when wet and a high follow-up property against twisting, and excellent absorption and leakage resistance even with small thicknesses.

However, the absorbent sheet of PTL 1 has a bulky structure wherein the base sheet is formed of paper comprising at least cellulose fiber, and voids are maintained by the skeletal structure formed by the fiber. Therefore, while the absorbent sheet of PTL 1 has increased wet strength by the use of heat-fusible binder fiber or a paper strength reinforcer, it has virtually no ability to recover from deformation, such as twisting or wrinkles when in a wet state.

With the highly absorbent complex sheet of PTL 2, the base sheet is a nonwoven fabric, and the fiber starting material used may be not only natural fiber, such as cellulose-based fiber but also synthetic fiber, such as a polyolefin or polyester. However, when cellulose-based fiber is used as the fiber starting material, the resulting absorbent sheet has virtually no ability to recover from deformation caused by twisting and wrinkles when wet, similar to PTL 1, and when synthetic fiber is used as the fiber starting material, it is resistant to deformation due to twisting and wrinkles but exhibits inferior fluid diffusibility and absorbed fluid retention volume, and therefore the utilization efficiency of the super-absorbent polymer is impaired, and fluid overflows before being absorbed by the super-absorbent polymer, tending to result in leakage.

Also, the absorbent body of PTL 3 has a polymer layer, comprising a super-absorbent polymer and a synthetic resin with excellent deformation stability, laminated on a layer comprising a fibrous base. Therefore, the same problems occur as with PTL 2, both when cellulose-based fiber is used as the fibrous base and when synthetic fiber is used as the fibrous base.

Citation List

Patent Literature

PTL 1 Japanese Unexamined Patent Publication No. 8-246395
PTL 2 Japanese Unexamined Patent Publication No. 2001-171027
PTL 3 Japanese Unexamined Patent Publication No. 10-118115

Summary of Invention

Technical Problem

As mentioned above, the absorbent sheets disclosed in PTLs 1-3 have not been able to exhibit both absorption properties including fluid retention, fluid diffusibility and absorbed fluid retention volume, and deformation resistance against deformation due to twisting and wrinkles.
It is therefore an object of the present invention to provide a layered absorbent sheet with excellent absorption properties including fluid retention, fluid diffusibility and absorbed fluid retention volume, and with particularly excellent deformation resistance against deformation by twisting and wrinkles.

Solution to Problem

As a result of diligent research directed toward solving the problems described above, the present inventors have found that the problems can be solved by a layered absorbent sheet comprising a first sheet layer on the absorbing side, a second sheet layer on the non-absorbing side, and a super-absorbent polymer layer between the first sheet layer and second sheet layer, wherein the first sheet and/or second sheet are bulky absorbent sheets, the bulky absorbent sheets comprising heat-expanding particles that are dispersed in the bulky absorbent sheet and have been expanded by heat, and the invention has been completed upon this finding.

Specifically, the present invention relates to the following aspects.

[Aspect 1]

A layered absorbent sheet comprising a first sheet layer on the absorbing side, a second sheet layer on the non-absorbing side, and a super-absorbent polymer layer between the first sheet layer and second sheet layer:

wherein the first sheet and/or second sheet are bulky absorbent sheets,
the bulky absorbent sheets comprising heat-expanding particles that are dispersed in the bulky absorbent sheet and have been expanded by heat.

[Aspect 2]

The layered absorbent sheet according to aspect 1, wherein the bulky absorbent sheet is a bulky absorbent sheet with a density of 0.025-0.1 g/cm$^3$, obtained by a production method comprising the steps of:

forming a wet blended sheet comprising the heat-expanding particles dispersed in the fiber starting material from a paper-making starting material in which a fiber starting material comprising 30-100 mass% of natural pulp and 0-70 mass% of other fiber, and heat-expanding particles with a mean particle size of 5-30 $\mu$m, are dispersed in water at a proportion of 5-30 parts by mass of the heat-expanding particles per 100 parts by mass of the fiber starting material, and then
expanding at least some of the heat-expanding particles by heat to a 20-125 fold volume, to obtain a bulky absorbent sheet.

[Aspect 3]

The layered absorbent sheet according to aspect 2, wherein the heating is carried out by moist hot air or water vapor, and the expanding step is further followed by a step of drying the wet blended sheet.

[Aspect 4]

The layered absorbent sheet according to any one of aspects 1 to 3, wherein the bulky absorbent sheet has a concavoconvex pattern consisting of one or more high-basis-weight regions and one or more low-basis-weight regions.

[Aspect 5]

The layered absorbent sheet according to aspect 4, wherein the one or more low-basis-weight regions are interspersed within the high-basis-weight region.

[Aspect 6]

The layered absorbent sheet according to any one of aspects 1 to 5, wherein the bulky absorbent sheet has a concavoconvex pattern consisting of one or more low density regions with a high degree of expansion of the heat-expanding particles, and one or more high density regions with a low degree of expansion of the heat-expanding particles.

[Aspect 7]

The layered absorbent sheet according to any one of aspects 1 to 6, wherein both the first sheet and the second sheet are bulky absorbent sheets.

[Aspect 8]

The layered absorbent sheet according to any one of aspects 1 to 7, wherein the first sheet is the bulky absorbent sheet, and the second sheet is water-resistant tissue paper.

[Aspect 9]

The layered absorbent sheet according to any one of aspects 1 to 8, which further comprises a fluff pulp layered mat layer on the non-absorbing side of the second sheet.

Advantageous Effects of Invention

Since the layered absorbent sheet of the invention has excellent fluid diffusibility, because voids are formed by the spaces between the expanded heat-expanding particles and the fibers and the spaces between the fibers, which voids are suitable for diffusion of fluids in the bulky absorbent sheet, the utilization efficiency of the super-absorbent polymer can be increased.
In addition, since the volume of the aforementioned voids in the bulky absorbent sheet is greater than that in a conventional absorbent sheet, such that the absorbed fluid retention volume is excellent, the stacked absorbing sheet of the invention can instantly absorb large amounts of excreted fluid.
The layered absorbent sheet of the invention also has excellent deformation resistance against deformation due to twisting and wrinkles, even when wet, because of the impact resilience of the heat-expanding particles that have been expanded in the bulky absorbent sheet, and exhibits excellent shape restoring properties whereby upon deformation, it is rapidly restored to its original shape.

Brief Description of Drawing

Fig. 1 is an electron micrograph showing the surface of an example of a bulky absorbent sheet used for the invention.
Fig. 2 is an electron micrograph showing a cross-section of an example of a bulky absorbent sheet used for the invention.
Fig. 3 is a schematic view of a paper machine that can produce a bulky absorbent sheet to be used for the invention.

Fig. 4 shows a plan view of a nozzle plate comprising jet holes and an oblique view of the jet nozzle incorporating it.
Fig. 5 shows a plan view of a nozzle plate comprising a spray slit and an oblique view of the jet nozzle incorporating it.
Fig. 6 shows wavy lines created by reciprocal movement of the jet nozzle in the CD direction.
Fig. 7 shows wavy lines created by reciprocal movement of a multi-level jet nozzle in the CD direction.
Fig. 8 is a schematic view of a paper machine that can produce a bulky absorbent sheet to be used for the invention.
Fig. 9 is a plan view of paper-making wire for obtaining a bulky absorbent sheet having multiple low-basis-weight regions interspersed within high-basis-weight region.
Fig. 10 is a plan view of paper-making wire for obtaining a bulky absorbent sheet having multiple high-basis-weight regions interspersed within low-basis-weight region.
Fig. 11 is a plan view of paper-making wire for obtaining a bulky absorbent sheet having multiple high-basis-weight regions and multiple low-basis-weight regions arranged as lines in an alternating fashion in one direction.
Fig. 12 shows an example of a bulky absorbent sheet produced by the paper machine shown in Fig. 8.
Fig. 13 is a schematic cross-sectional view showing a variation example of the layered absorbent sheet of the invention.
Fig. 14 is a drawing showing a bonding example of the layered absorbent sheet of the invention.
Fig. 15 is a drawing showing an example of an absorbent article employing the layered absorbent sheet of the invention.
Fig. 16 is a schematic cross-sectional view showing a variation example of an absorbent article employing the layered absorbent sheet of the invention.
Fig. 17 is a schematic diagram showing an apparatus that can produce an absorbent article employing the layered absorbent sheet of the invention.
Fig. 18 is a schematic diagram showing an apparatus that can produce the layered absorbent sheet of the invention.
Fig. 19 is a diagram illustrating a method of measuring the width compression recoverability.

Description of Embodiments

The present invention will now be explained in greater detail, with the understanding that the invention is not limited to this explanation.

[First sheet and second sheet]

[Bulky absorbent sheet]

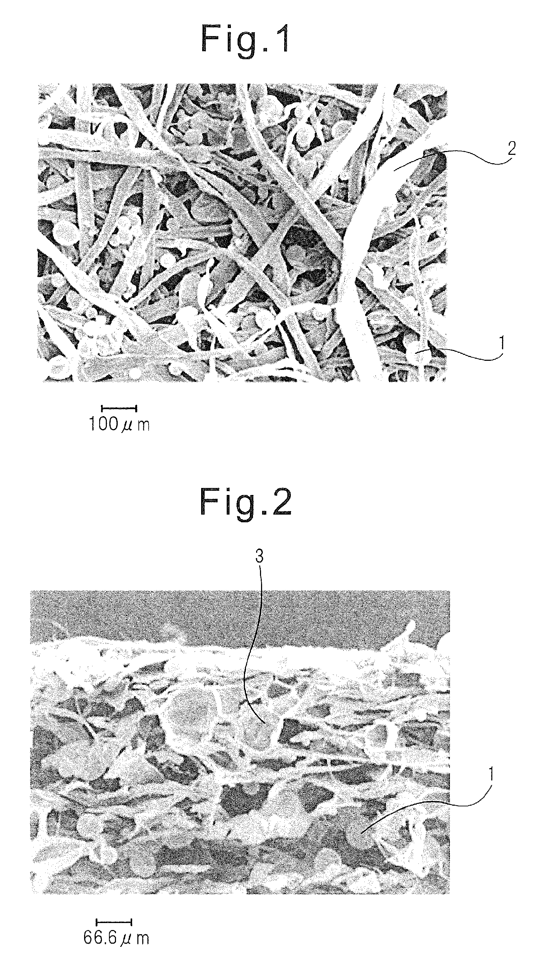

The bulky absorbent sheet composing the first sheet and/or second sheet comprises heat-expanding particles that are dispersed in the bulky absorbent sheet, and are expanded by heat.
The bulky absorbent sheet used for the invention is a sheet in which a plurality of expanded heat-expanding particles are dispersed in an absorbent sheet, and it differs from a bulky structure formed by maintaining spaces by the coupled structure of the fibers themselves, as in a conventional bulked sheet. Thus, even when a load, such as compression or bending is applied to the bulky absorbent sheet in a wet state, the expanded heat-expanding particles exhibit elasticity against the load so that they are resistant to deformation, and the original shape can be maintained. Consequently, little of the fluid that has been absorbed is squeezed out by subsequent deformation, such as compression. Even if deformation has occurred by an excessive load, the elasticity allows it to easily be restored to its original shape.
In addition, the expanded heat-expanding particles are formed by thermal expansion after the non-expanded heat-expanding particles have been dispersed in the absorbent sheet, and therefore spaces are formed between the fibers by expansion of the heat-expanding particles. Thus, the spaces between the expanded heat-expanding particles and the fibers and the spaces between the fibers form voids suitable for diffusion of fluid, and can provide both of the reciprocal properties of bulk and fluid diffusibility.
If a bulky absorbent sheet having such excellent fluid diffusibility is situated adjacent to a super-absorbent polymer layer, fluid that has been absorbed by the bulky absorbent sheet can be rapidly absorbed into the super-absorbent polymer layer while being rapidly diffused into the bulky absorbent sheet.
Electron micrographs of the surface and cross-section of an example of a bulky absorbent sheet are shown in Fig. 1 and Fig. 2, respectively. In Fig. 1 and Fig. 2, numeral 1 denotes an expanded expanding particle, numeral 2 denotes the fiber starting material, and numeral 3 denotes the cross-section of an expanded heat-expanding particle.
The heat-expanding particles used for the invention are heat-expanding microcapsules obtained by encapsulating a low-boiling-point solvent in microcapsules formed from a film polymer. There are no particular restrictions on the particle size of the heat-expanding particles, but in consideration of the fluid absorption rate, fluid diffusibility, absorbed fluid retention volume, fluid retention and twisting resistance of the bulky absorbent sheet, the mean particle size before expansion is preferably 5-30 μm and more preferably 8-14 μm. The low-boiling-point solvent generates a gas, by volatilization, for example, at a lower temperature than the softening temperature, such as a temperature below the glass transition temperature, of the film polymer.

As used herein, "mean particle size" means the particle size at 50% in the volume-based cumulative distribution, of the particle size distribution measured with a laser diffraction particle size distribution analyzer (for example, HEROS & RODOS by JEOL Corp.).

Also, the term "heat-expanding particles", as used herein, means particles before expansion, while "expanded heat-expanding particles" means the particles after expansion by heat or the like.

The heat-expanding particles expand preferably 20-to 125-fold and more preferably 50- to 80-fold by volume upon brief heating at a relatively low temperature of 80-200°C. If the volume expansion is less than 20-fold, spaces will not easily form between the fibers, and the fluid absorption rate, fluid diffusibility and absorbed fluid retention volume of the bulky absorbent sheet will tend to be inadequate. If the volume expansion is greater than 125-fold, the fluid diffusibility of the bulky absorbent sheet will tend to be insufficient, and the elasticity of the expanded expanding particles will tend to be reduced, leading to more twisting and flattening.

The low-boiling-point solvent may be a volatile organic solvent (expanding agent), such as isobutane, pentane, petroleum ether, hexane, a low-boiling-point halogenated hydrocarbon, methylsilane, or the like.

The film polymer may be a thermoplastic resin composed of a copolymer of vinylidene chloride, acrylonitrile, acrylic acid ester, methacrylic acid ester or the like, and upon heating the film polymer at above the softening point, the film polymer begins to soften causing the vapor pressure of the encapsulated low-boiling-point solvent to increase simultaneously, so that the film is pushed outward resulting in expansion of the capsules. The heat-expanding particles expand at relatively low temperature and in a short period of time to form closed cells, thus creating particles with excellent elasticity and relatively easy manageability, which are suitable for the present purpose.

As such heat-expanding particles there are known Matsumoto Microspheres F-36, F-30D, F-30GS, F-20D, F-50D and F-80D (products of Matsumoto Yushi-Seiyaku Co., Ltd.) and EXPANCEL WU and DU (products of Sweden, marketed by Japan Fillite Co., Ltd.), although there is no limitation to these.

The fiber starting material used for the invention may be any one commonly used in the field of paper-making, without any particular restrictions, and examples include natural pulp, synthetic pulp, organic fiber and inorganic fiber. The fiber starting material preferably comprises 30-100 mass% of natural pulp and 0-70 mass% of other fiber, and more preferably it comprises 50 mass%-100 mass% of natural pulp and 0-50 mass% of other fiber. This will result in excellent anchoring, yield and uniform dispersibility of the heat-expanding particles, as well as excellent uniformity and strength of the sheet.

The other fibers are preferably selected from the group consisting of synthetic pulp, organic fibers and inorganic fibers. The other fibers used may be, for example, synthetic fibers with a low melting point, when a heat sealing property is to be imparted to the bulky absorbent sheet used for the invention, or combined fibers with a long fiber length (3-25 mm), when breaking resistance is to be imparted.

The natural pulp may be wood pulp, such as chemical pulp or mechanical pulp from a conifer and/or broadleaf tree, waste paper pulp, or nonwood natural pulp, such as hemp or cotton, though there is no restriction to these. As synthetic pulp there may be mentioned synthetic pulp obtained from polyethylene or polypropylene starting materials, though there is no limitation to these. As the organic fiber there may be mentioned acrylic fiber, rayon fiber, phenol fiber, polyamide fiber and polyethylene fiber, with no limitation to these. As the inorganic fiber there may be mentioned glass fiber, carbon fiber, alumina fiber and the like, with no limitation to these.

The preferred amount of the heat-expanding particles will vary depending on the purpose of use and is not particularly restricted, but generally speaking it is preferably 5-30 parts by mass, more preferably 8-25 parts by mass and even more preferably 10-15 parts by mass, with respect to 100 parts by mass of the fiber starting material. An amount of heat-expanding particles of less than 5 parts by mass with respect to 100 parts by mass of the fiber starting material will result in less expansion, while an amount of greater than 40 parts by mass will tend to be economically disadvantageous.

The preferred value for the density of the bulky absorbent sheet used for the invention will differ depending on the purpose of use and is not particularly restricted, but generally speaking the sheet preferably has a density of 0.025-0.1 g/cm$^3$ and more preferably a density of 0.03-0.07 g/cm$^3$. If the density is less than 0.025 g/cm$^3$ the wet strength will be reduced or the proportion of heat-expanding particles will be increased, which is economically disadvantageous, while if the density is greater than 0.1 g/cm$^3$ the absorbed fluid retention volume and stability against twisting deformation will tend to be inferior.

An anchoring agent which is commonly used in the paper-making industry may be added during production of the bulky absorbent sheet to be used for the invention, in order to anchor the heat-expanding particles to the fibers.

In order to increase the wet sheet strength, an internal wet strength agent commonly used in the paper-making industry may be added during production, in addition to the low-melting-point synthetic fiber. By adding a wet strength agent it is possible to increase the water resistance of the bulky absorbent sheet.

However, excessive addition of the aforementioned anchoring agent or wet strength agent may result in inhibition of hydrophilicity and loss of flexibility of the bulky absorbent sheet, and therefore the addition amounts must be adjusted according to the purpose.

If desired, various anionic, nonionic, cationic or amphoteric yield improvers, paper strength additives, sizing agents and the like may also be added to the bulky absorbent sheet to be used for the invention. Specifically, as paper strength additives and yield improvers there may be used organic compounds, such as polyacrylamide-based cationic, nonionic, anionic and amphoteric resins, polyethyleneimine and its derivatives, polyethylene oxide, polyamines, polyamides, polyamidepolyamine and its derivatives, cationic and amphoteric starch, oxidized starch, carboxymethylated starch, vegetable gum, polyvinyl alcohol, urea-formalin resin, melamine-formalin resin and hydrophilic polymer particles, and inorganic compounds including aluminum compounds, such as aluminum sulfate, alumina sol, basic aluminum sulfate, basic aluminum chloride and basic polyaluminum hydroxide, and ferrous sulfate, ferrous chloride, colloidal silica, bentonite or the like, either alone or in combinations.

Production of the bulky absorbent sheet to be used for the invention is not particularly restricted, but it may be accomplished in the following manner, for example. [paper-making step]

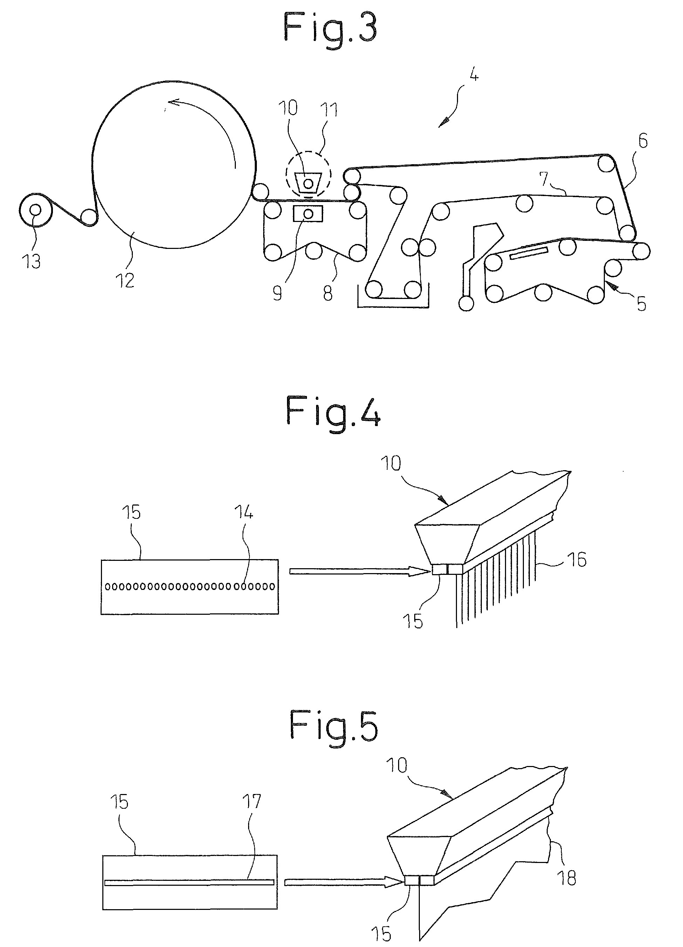

Fig. 3 is a schematic view of a paper machine that can produce a bulky absorbent sheet to be used for the invention. The paper machine 4 comprises a paper-making part 5, a wet blended sheet 6, a first conveyor belt 7, a second conveyor belt 8, a suction box 9, a jet nozzle 10, a screen 11, a dryer 12 and a finished product take-up roll 13. The wet blended sheet 6 is made by the paper-making part 5 from a paper-making starting material comprising a fiber starting material and heat-expanding particles dispersed in water, and the wet blended sheet 6 is conveyed by the first conveyor belt 7 and then dewatered by a press part.

In common paper-making steps, the water content of the wet blended sheet is adjusted to about 60 mass% by the dehydration step.

[Thermal expansion step]

The dewatered wet blended sheet is conveyed by the second conveyor belt 8, and heated by spraying of hot air or the like from the jet nozzle 10 to expand the heat-expanding particles. If the dewatered wet blended sheet is placed on a support and suction is applied from the suction box 9 from the bottom side of the support while spraying the hot air from the top side, the entire sheet will be heated rapidly and evenly, thereby increasing the thermal expansion effect and raising efficiency. The support may be, but is not limited to, a net or other type of conveyor belt.

[Drying step]

If desired, the sheet may be dried by the dryer 12 and the bulky absorbent sheet taken up on the finished product take-up roll 13. Expansion of the heat-expanding particles and drying of the bulky absorbent sheet may be accomplished simultaneously with the dryer 12, while eliminating spraying of hot air or the like from the jet nozzle 10.

As a separate aspect for production of a bulky absorbent sheet to be used for the invention, moist hot air or water vapor at a prescribed temperature may be sprayed from the jet nozzle 10 to heat and expand the heat-expanding particles without drying the wet blended sheet. By using moist hot air or water vapor, the dewatered wet blended sheet does not dry even when excess heat is applied, for example, and it is possible to adequately expand the heat-expanding particles since no bonding strength is produced between fibers that would inhibit expansion of the heat-expanding particles. After the heat-expanding particles have been adequately expanded, the bulky absorbent sheet may be dried with the dryer 12.

When the thermal expansion step described above is accomplished by heating the dewatered wet blended sheet with moist hot air or water vapor at a prescribed temperature, the wet blended sheet is preferably dewatered to a low water content of, for example, 40-60 mass% in the dehydration step, in order to efficiently increase the temperature of the wet blended sheet as a whole to the prescribed temperature.

The temperature of the warm air, moist hot air or water vapor in the thermal expansion step is above the temperature at which the microcapsule shell walls of the heat-expanding particles soften and begin to expand, and it is a temperature determined by the heat-expanding particles used.

The humidity of the moist hot air or water vapor is preferably a high relative humidity, such as 100% RH, when the wet blended sheet is not dried in the thermal expansion step. The means for supplying the moist hot air or water vapor is most preferably means in which high-temperature steam from a boiler is ejected and directly sprayed onto the sheet, but humidified exhaust from a drier may also be used.

- Bulky absorbent sheet with concavoconvex pattern comprising one or more low density regions and one or more high density regions -

A bulky absorbent sheet having a concavoconvex pattern comprising low one or more density regions with a high degree of expansion of heat-expanding particles and one or more high density regions with a low degree of expansion of heat-expanding particles may be produced by spraying prescribed sections of a wet blended sheet with moist hot air or water vapor at above the initial expansion temperature of the heat-expanding particles to cause expansion of the heat-expanding particles at those sections, and subsequently drying at a temperature at which the heat-expanding particles do not fully expand.

As an example of a jet nozzle that can produce a bulky absorbent sheet having a concavoconvex pattern with one or more low density regions and one or more high density regions, Fig. 4 shows a plan view of a nozzle plate 15 with jet holes 14 and an oblique view of a jet nozzle 10 incorporating the same. Using the nozzle plate 15 shown in Fig. 4 produces a columnar jet 16.

As another example of the aforementioned jet nozzle, Fig. 5 shows a plan view of a nozzle plate 15 comprising a spray slit 17, and an oblique view of a jet nozzle 10 incorporating it. Using the nozzle plate 15 shown in Fig. 5 produces a curtain jet 18.

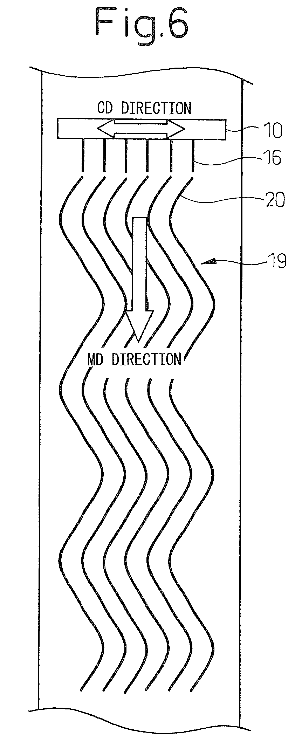

When the jet nozzle 10 shown in Fig. 4 is used to spray moist hot air or water vapor onto a wet blended sheet by a columnar jet 16, the jet nozzle 10 may be fixed, or the jet nozzle 10 may be reciprocally moved in the CD direction of the wet blended sheet 1, to create a concavoconvex pattern of wavy lines 20 extending in the MD direction, as shown in Fig. 6.

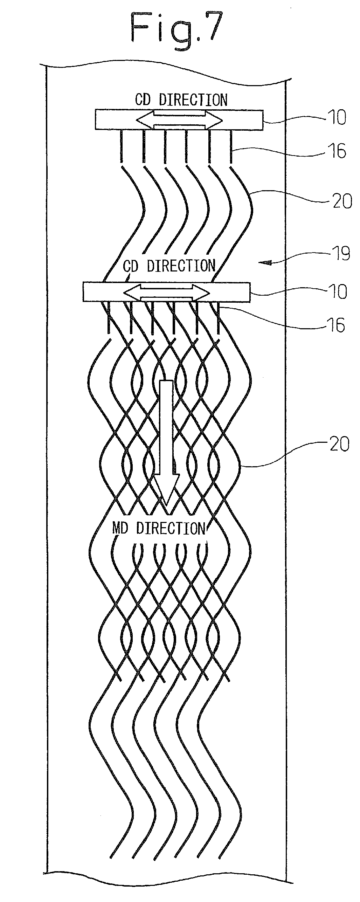

Fig. 7 shows wavy lines created by reciprocal movement of a multi-level jet nozzle in the CD direction. If multiple jet nozzles 10 are provided as shown in Fig. 7, it is possible to form an interlaced pattern with crossed wavy lines 20. The pitch and heights of the waves are determined by the number of strokes (per minute) of the jet nozzle in the CD direction and the running speed (m/min) of the sheet in the MD direction. If multiple jet nozzles are provided, various different patterns can be produced by altering the stroke distance and cycle for each of the jet nozzles.

As used herein, "MD direction" means the machine direction during production, and "CD direction" means the cross machine direction perpendicular to the machine direction.

The density in the low density regions of the bulky absorbent sheet used for the invention is preferably 0.01 g/cm$^3$-0.1 g/cm$^3$ and more preferably 0.01 g/cm$^3$-0.05 g/cm$^3$, and the density in the high density regions is preferably 0.1 g/cm$^3$-0.3 g/cm$^3$.

When the bulky absorbent sheet has one or more low density regions and one or more high density regions, the term "density" used alone will refer to the apparent density of the bulky absorbent sheet.

If the density in the low density regions of the bulky absorbent sheet used for the invention is greater than 0.1 g/cm$^3$, the fluid retention property, deformation resistance and deformation stability will be reduced, while if it is less than 0.01 g/cm$^3$ the strength will be reduced and tearing will easily occur, tending to cause problems with surface friction durability. If the density of the high density regions of the bulky absorbent sheet is less than 0.1 g/cm$^3$ the fluid diffusibility will be impaired, and if it exceeds 0.3 g/cm$^3$, the heat-expanding particles will be in a virtually unexpanded state, such that absolutely none of the properties from the bulky absorbent sheet will be obtained.

- Bulky absorbent sheet with concavoconvex pattern comprising one or more low-basis-weight regions and one or more high-basis-weight regions -

A bulky absorbent sheet having a concavoconvex pattern comprising partial low-basis-weight regions with a low paper-making starting material content and partial high-basis-weight regions with a high paper-making starting material content, can be produced by using a partially blocked wire to obtain a wet blended sheet comprising partial low-basis-weight regions with a low paper-making starting material content and partial high-basis-weight regions with a high paper-making starting material content.

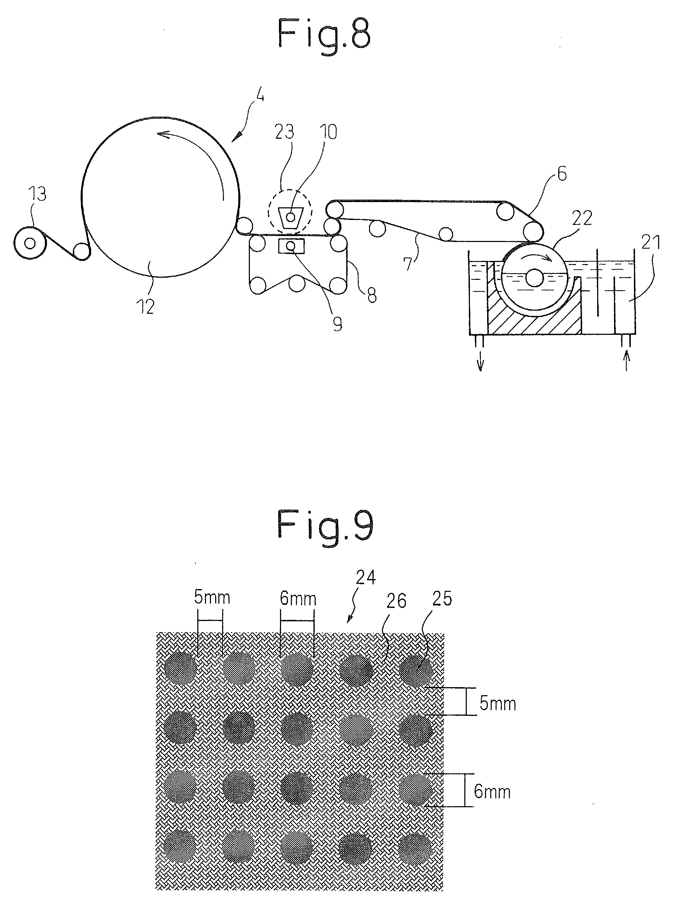

To produce a bulky absorbent sheet having a concavoconvex pattern comprising one or more low-basis-weight regions and one or more high-basis-weight regions, there may be used the paper machine illustrated in Fig. 8 and a partially blocked paper-making wire 24, such as shown in Figs. 9 to 11. When a partially blocked wire is used, it is possible to obtain a wet blended sheet comprising partial low-basis-weight regions with a low paper-making starting material content and partial high-basis-weight regions with a high paper-making starting material content. Specifically, water penetrates poorly at the blocked sections and therefore the paper-making starting material does not easily accumulate, thus forming partial low-basis-weight regions with a low paper-making starting material content, while water penetrates readily at the non-blocked sections and therefore the paper-making starting material easily accumulates, thus forming partial high-basis-weight regions with a high paper-making starting material content.

As used herein, partial regions with a low paper-making starting material content and a lower basis weight than the average basis weight will be referred to as "a low-basis-weight region", and partial region with a high paper-making starting material content and a higher basis weight than the average basis weight will be referred to as "a high-basis-weight region". If the heat-expanding particles are evenly dispersed in the paper-making starting material as according to the invention, the heat-expanding particles will be present in about the same proportion in the low-basis-weight regions and high-basis-weight regions, so that heating will cause expansion to produce bulk in both by the same proportion. The apparent bulk of the paper in the high-basis-weight regions having a higher basis weight than the average basis weight is larger than the average bulk, while the low-basis-weight regions are the opposite. It is therefore possible to obtain a bulky absorbent sheet with high apparent bulk in a large concavoconvex pattern.

The paper machine 4 of Fig. 8 comprises a paper-making starting material solution 21, a paper-making cylinder 22, a wet blended sheet 6, a first conveyor belt 7, a second conveyor belt 8, a suction box 9, a jet nozzle 10, a screen drum 23, a dryer 12 and a finished product take-up roll 13. A paper-making wire 24 is also mounted on the paper-making cylinder 22.

The paper-making cylinder 22 and paper-making wire 24 are used to produce a wet blended sheet 6 comprising one or more high-basis-weight regions and one or more low-basis-weight regions from a paper-making starting material solution 21 obtained by dispersing a fiber starting material and heat-expanding particles in water, wherein the wet blended sheet 6 is conveyed by a first conveyor belt 7 and a second conveyor belt 8, the wet blended sheet 6 is subsequently heated by hot air, moist hot air or water vapor from the jet nozzle 10 to cause expansion of the heat-expanding particles, the sheet is then dried if desired with the dryer 12, and the finished bulky absorbent sheet is taken up with the finished product take-up roll 13 to obtain a bulky absorbent sheet with a concavoconvex pattern.

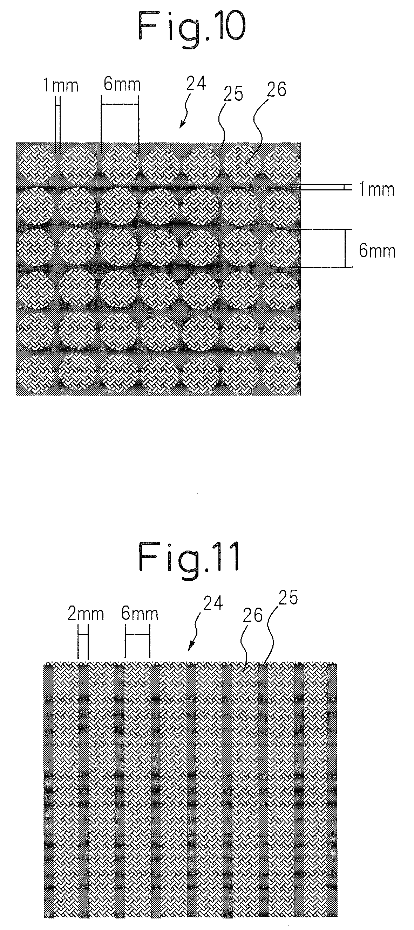

Blocking of the paper-making wire 24 can be accomplished using a reaction curing resin or the like, and the sizes, number, shapes and arrangement thereof may be freely designed. For example, as shown in Fig. 9, the blocked regions 25 may be interspersed in the non-blocked regions 26, as shown in Fig. 10, the non-blocked regions 26 may be interspersed in the blocked regions 25, or as shown in Fig. 11, linear blocked regions 25 and linear non-blocked regions 26 may be arranged in an alternating fashion.

Low-basis-weight regions do not form as easily with a smaller single blocking size, while low-basis-weight regions form more easily at larger sized sections. If the size of a single blocked region is too small, the blocked sections will become covered with the paper-making starting material, filling in the blocked sections and thus preventing formation of low-basis-weight regions. On the other hand, if the size of a single blocked region is too large, uniform low-basis-weight regions will not form but rather open sections without paper-making starting material will tend to be created, resulting in easier tearing at these open sections during movement from the paper-making wire to the conveyor belt, thus impeding movement.

The optimum range for the size of a single blocked region cannot be specified since it will vary depending on the basis weight of the sheet. The area ratio of the blocked sections with respect to the total wire may be varied as necessary, but a larger area ratio is more effective for improving the apparent bulk of the sheet, whereas a smaller one reduces the apparent bulk. If the area ratio is too large, the starting material will concentrate excessively at the non-blocked sections during paper making, thus interfering with production of the sheet. The area ratio of the blocked sections with respect to the total wire will vary depending on the shapes of the blocked regions, but it may be 10%-60% and is preferably 20%-50%.

In the paper-making wire 24 shown in Fig. 9, for example, the blocked regions 25 may be circles with diameters of 6 mm, and the distance between each blocked region 25 may be 5 mm. The remaining sections are the non-blocked regions 26. In the paper-making wire 24 shown in Fig. 10, for example, the non-blocked regions 26 may be circles with diameters of 6 mm, and the distance between each non-blocked region 26 may be 1 mm. The remaining sections are the blocked regions 25. In the paper-making wire 24 shown in Fig. 11, for example, the blocked regions 25 may be rectangles with widths of 2 mm, and the distance between each blocked region 25 may be 6 mm. The remaining sections are the non-blocked regions 26.

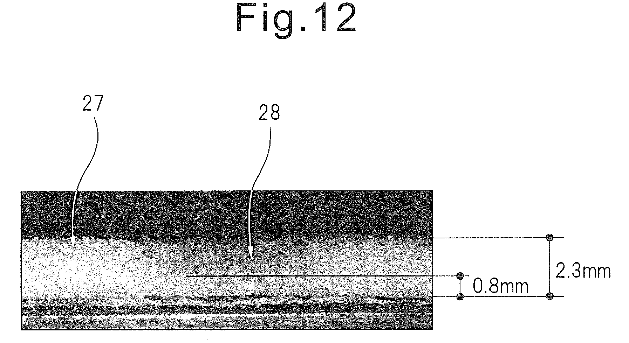

Fig. 12 shows an example of a bulky absorbent sheet produced by the paper machine 4 shown in Fig. 8. The bulky absorbent sheet produced by the paper machine 4 shown in Fig. 8 has high-basis-weight regions 27 and low-basis-weight regions 28.

The low-basis-weight region has rapid fluid penetration, and therefore when it instantly receives a large amount of body fluid, the fluid does not flow over the absorbent sheet surface but penetrates through to the lower layer. Since the one or more low-basis-weight regions are dispersed among the high-basis-weight region, there is essentially no reduction in the sheet strength as a whole. By providing one or more low-basis-weight regions, it is possible to further increase the basis weight of the one or more high-basis-weight regions, further increase the thickness of the one or more high-basis-weight regions compared to homogeneity, and to produce an excellent effect of preventing return of fluids and excellent cushioning properties. In consideration of the fluid permeation effect, the size of each low-basis-weight region is preferably such that the diameter or side length is 1 mm-5 mm. If it is less than 1 mm, the resistance will be too great during fluid permeation, and if it exceeds 5 mm, fluid will tend to flow back from the lower layer.

When a concavoconvex pattern is imparted to a bulky water-absorbing sheet, as in a bulky absorbent sheet with a concavoconvex pattern comprising low one or more density regions and one or more high density regions, or a bulky absorbent sheet with a concavoconvex pattern comprising one or more low-basis-weight regions and one or more high-basis-weight regions, the super-absorbent polymer particles can be concentrated at the recesses, and distribution of the super-absorbent polymer particles is simplified. In the case of a concavoconvex pattern comprising one or more low density regions and one or more high density regions, the super-absorbent polymer that has been distributed in a concentrated manner at the convex low density regions by suction falls by gravity into the concave high density regions after passing through the suction zone. In the case of a concavoconvex pattern comprising one or more low-basis-weight regions and one or more high-basis-weight regions, it is distributed in a concentrated manner at the concave low-basis-weight regions by suction.

When the super-absorbent polymer is distributed in a concentrated manner at the recesses, bonding between the first sheet and second sheet can be accomplished mainly at the heights which have a low super-absorbent polymer content, and therefore integral bonding is facilitated. Also, a low super-absorbent polymer concentration at the heights will prevent the blocking phenomenon by swelling of the super-absorbent polymer.

[Other sheet-like material]

Either or both the first sheet and second sheet are the aforementioned bulky absorbent sheet, while sheets other than the bulky absorbent sheet may be selected from among other sheet-like materials, depending on the purpose. Examples for the other sheet-like material include water-resistant tissue paper and fluff pulp layered mats.

As used herein, "water-resistant tissue paper" refers to tissue paper with a moist tensile strength of 0.6 N/25 mm or greater in both the MD direction and CD direction. The moist tensile strength can be measured according to JIS P 8135. Also, as used herein, "fluff pulp layered mat" refers to a mat in which a fluff pulp is laminated.

[Super-absorbent polymer]

As used herein, "super-absorbent polymer" refers to a synthetic polymer-based absorber, such as a starch-based, cellulose-based, polyacrylic acid-based, polyvinyl alcohol-based, polyacrylamide-based or polyoxyethylene-based absorber. The super-absorbent polymer is preferably a polyacrylic acid-based absorber and more preferably a sodium polyacrylate-based absorber.

[Layered absorbent sheet]

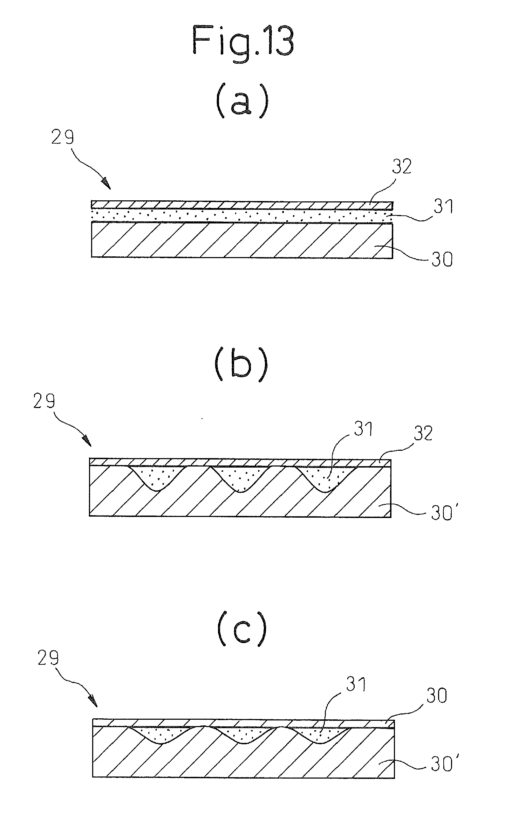

The layered absorbent sheet of the invention may be selected from various forms, depending on the purpose. Fig. 13 is a schematic cross-sectional view showing a variation example of the absorbent sheet of the invention. The layered absorbent sheet 29 of the invention may have water-resistant tissue paper 32 as the first sheet and a bulky absorbent sheet 30 as the second sheet, as shown in Fig. 13(a), for example. Alternatively, the first sheet may be a bulky absorbent sheet 30 and the second sheet the water-resistant tissue paper 32.

As used herein, "first sheet" refers to the sheet situated on the fluid-absorbing side, adjacent to the super-absorbent polymer layer. Also, as used herein, "second sheet" refers to the sheet situated on the side opposite the first sheet side, adjacent to the super-absorbent polymer layer. The second sheet is situated on the non-fluid-absorbing side.

The layered absorbent sheet 29 of the invention may also have water-resistant tissue paper 32 as the first sheet and a bulky absorbent sheet 30' with a concavoconvex pattern as the second sheet, as shown in Fig. 13(b), for example. Alternatively, the first sheet may be a bulky absorbent sheet 30' with a concavoconvex pattern and the second sheet the water-resistant tissue paper 32.

The layered absorbent sheet 29 of the invention may also have a bulky absorbent sheet 30 as the first sheet and a bulky absorbent sheet 30' with a concavoconvex pattern as the second sheet, as shown in Fig. 13(c), for example. Alternatively, the first sheet may be a bulky absorbent sheet 30' with a concavoconvex pattern and the second sheet the bulky absorbent sheet 30.

While not shown in Fig. 13, the first sheet or second sheet may instead be a fluff pulp layered mat.

When both the first sheet and second sheet are bulky absorbent sheets, the two bulky absorbent sheets may have different densities and basis weights, depending on the desired function.

In the schematic diagrams of Fig. 13(b) and (c), the super-absorbent polymer 31 is shown as being divided into multiple sections, but Fig. 13 is only a schematic diagram, and in actuality, the super-absorbent polymer 31 will usually also be present on the heights of the bulky absorbent sheet 30' with the concavoconvex pattern. Therefore, the super-absorbent polymer layer may be a flat sheet as shown in Fig. 13(a), or it may be a sheet with concavoconvexities as shown in Fig. 13(b) and (c) and with some open holes, or partially discontinuous sections may also be formed.

The first sheet in the layered absorbent sheet of the invention is most preferably the bulky absorbent sheet described above. This will allow the properties, and especially the fluid diffusibility, of the bulky absorbent sheet to be exhibited.

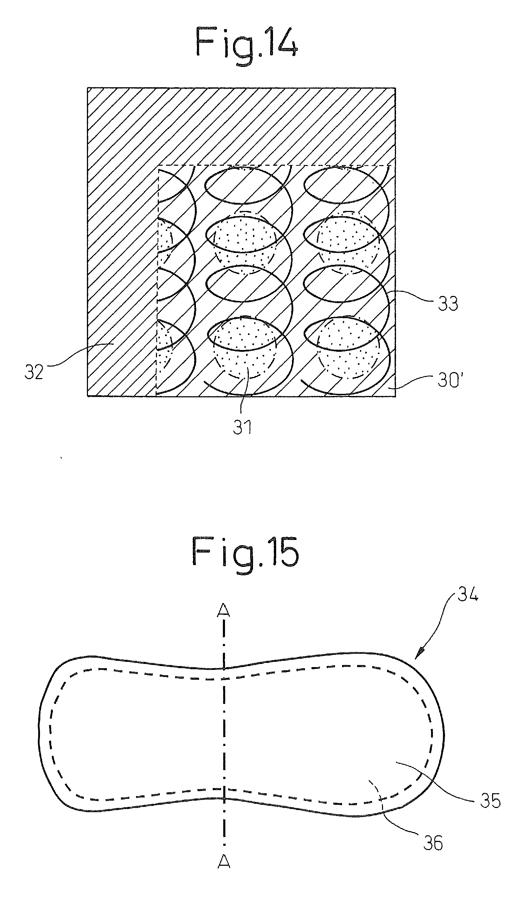

The layering means used for the layered absorbent sheet of the invention may be any means used in the technical field without any particular restrictions, and for example, it may be layered by bonding the first sheet and second sheet with a hot-melt adhesive, so as to sandwich the super-absorbent polymer between them. Fig. 14 shows a bonding example of the layered absorbent sheet of Fig. 13(b). In Fig. 14, the hot-melt adhesive 33 is coated in a spiral manner on the bulky absorbent sheet 30' with a concavoconvex pattern as the first sheet and the super-absorbent polymer 31, and a water-resistant tissue paper 32 layer is laminated as a second sheet on the hot-melt adhesive 33.

The hot-melt adhesive is not particularly restricted, and may be a hot-melt adhesive commonly employed in the technical field including, for example, polyolefin (such as polyethylene and polypropylene)-based hot-melt adhesives, ethylene/vinyl acetate copolymer-based hot-melt adhesives, and synthetic rubber (such as styrene-based polymer, butadiene-based polymer and isoprene-based polymer)-based hot-melt adhesives.

When a bulky absorbent sheet having a concavoconvex pattern comprising one or more low-basis-weight regions and one or more high-basis-weight regions is selected as the first sheet or second sheet of the layered absorbent sheet of the invention, and air is suctioned from the bottom side of the bulky absorbent sheet during dispersion of the super-absorbent polymer, through-air is concentrated at the recesses and the super-absorbent polymer becomes concentrated at the recesses. If the polymer particles are situated in the recesses, fly-off of the polymer during the polymer sheet production steps will be minimized.

Also, when a bulky absorbent sheet having a concavoconvex pattern comprising one or more low-basis-weight regions and one or more high-basis-weight regions is selected as the first sheet, the low-basis-weight recesses have excellent liquid-permeability. Consequently, fluid easily permeates initially from the recesses during use. Because the super-absorbent polymer is concentrated at the recesses, the permeated fluid can be effectively absorbed by the super-absorbent polymer.

The absorbed fluid permeates from the recesses while passing through the high-basis-weight heights and being dispersed, and therefore the super-absorbent polymer at sections other than those that have received the body fluid can efficiently absorb the fluid.

[Absorbent article]

In an absorbent article comprising a front sheet on the skin contact side, a back sheet on the non-skin contact side and an absorbent body situated between the two sheets, the layered absorbent sheet of the invention can be used as at least part of the absorbent body.

[Front sheet and back sheet]

Examples for the front sheet include known nonwoven fabrics, such as through-air nonwoven fabrics, point bond nonwoven fabrics, spunlace nonwoven fabrics, spunbond nonwoven fabrics, spunbond/meltblown/spunbond nonwoven fabrics and meltblown nonwoven fabrics, and liquid-permeable materials, such as porous films.

Examples for the back sheet include non-water-permeable film materials produced from polyethylene or polypropylene resins, moisture-permeable film materials containing inorganic filler materials, and water-permeable sheet materials used in the front sheet.

As used herein, "skin-contacting surface" refers to the side that contacts the skin during wearing, while "non-skin-contacting surface" refers to the surface on the side that does not contact the skin during wearing, which is, for example, the side that contacts underwear if the absorbent article is a sanitary napkin.

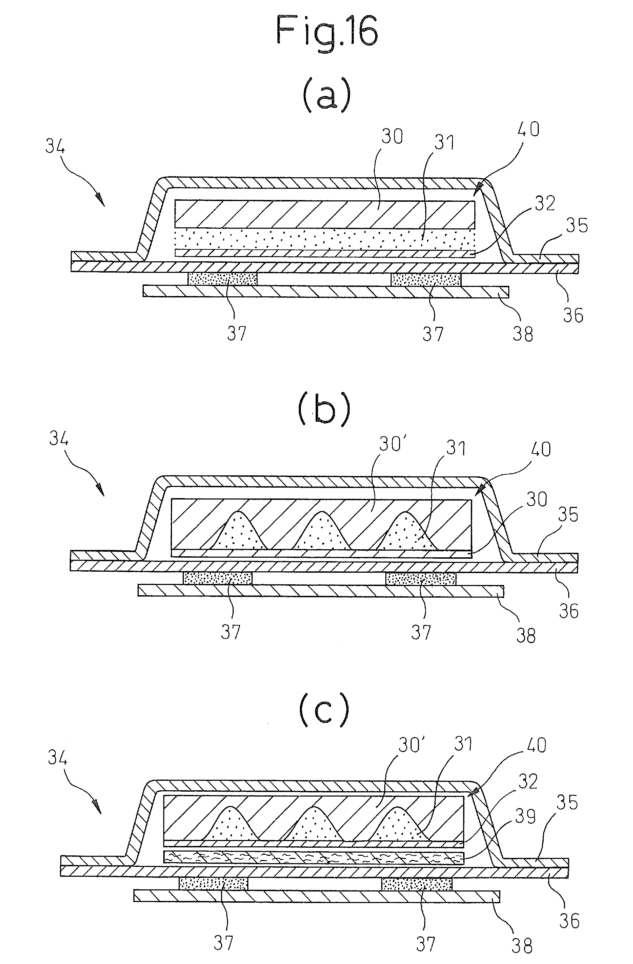

Fig. 16 is a schematic cross-sectional view showing a variation of an absorbent article employing the layered absorbent sheet of the invention. Fig. 16 is a cross-section of the absorbent article shown in Fig. 15, along direction A-A. The absorbent article 34 shown in Fig. 16 comprises a front sheet 35 on the skin contact side, a back sheet 36 on the non-skin contact side, and a layered absorbent sheet 40 positioned between these two sheets. The absorbent article 34 shown in Fig. 16 also has a cohesive part 37 and a detached portion 38 on the non-skin contact side of the back sheet 36. When the absorbent article is a sanitary napkin, for example, the absorbent article 34 may be anchored to underwear by the cohesive part 37.

In Fig. 16(a), the layered absorbent sheet 40 is composed of an absorbent sheet comprising a bulky absorbent sheet 30 layer as the first sheet layer, a super-absorbent polymer 31 layer, and a water-resistant tissue paper 32 layer as the second sheet layer.

In Fig. 16(b), the layered absorbent sheet 40 is composed of an absorbent sheet comprising a bulky absorbent sheet 30' layer with a concavoconvex pattern as the first sheet layer, a super-absorbent polymer 31 layer, and a bulky absorbent sheet 30 layer as the second sheet layer.

In Fig. 16(c), the layered absorbent sheet 40 is composed of an absorbent sheet, comprising a bulky absorbent sheet 30' layer with a concavoconvex pattern as the first sheet layer, a super-absorbent polymer 31 layer and a water-resistant tissue paper 32 layer as the second sheet layer, and a fluff pulp layered mat 39 layer.

If the layer of the layered absorbent sheet 40 adjacent to the front sheet 35 is the bulky absorbent sheet 30 layer or the bulky absorbent sheet 30' layer with a concavoconvex pattern, the bulky absorbent sheet which has an excellent fluid absorption rate and fluid diffusibility will rapidly absorb fluid, and will be able to rapidly and uniformly deliver it to the super-absorbent polymer 31 layer which is superior in terms of absorbed fluid retention volume and fluid retention, but has a low absorption rate and tends to produce the gel blocking phenomenon.

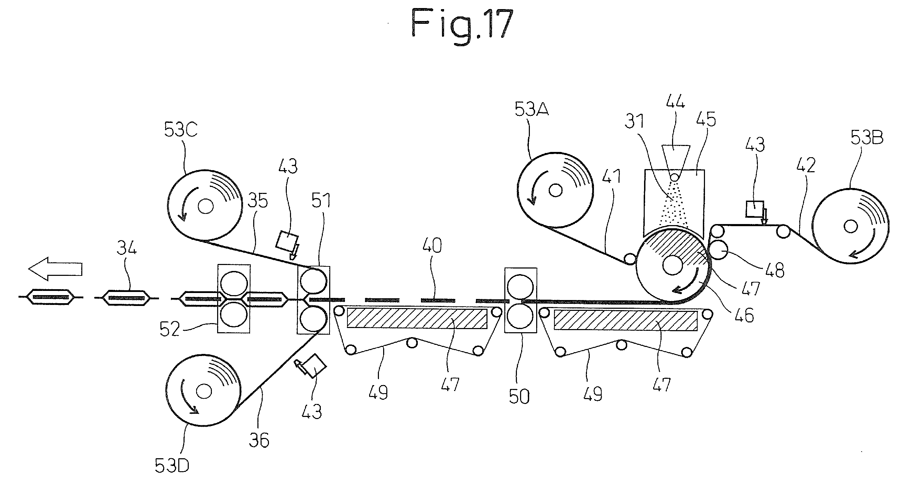

Fig. 17 is a schematic diagram showing an example of an apparatus that can produce a layered absorbent sheet of the invention and an absorbent article employing it as an absorbent body. The apparatus in Fig. 17 comprises a first sheet 41, a second sheet 42, a plurality of hot-melt adhesive coating machines 43, a super-absorbent polymer applicator 44, a super-absorbent polymer application chamber 45, a suction rotary drum 46, a plurality of suction zones 47, a first attachment roll 48, a plurality of belt conveyors 49, a front sheet 35, a back sheet 36, a layered absorbent sheet cutter 50, second attachment rolls 51, a product cutter 52 and rolls 53A, 53B, 53C, 53D.

In the apparatus shown in Fig. 17, the super-absorbent polymer applicator 44 and super-absorbent polymer application chamber 45 are used to apply the super-absorbent polymer 31 onto the first sheet 41 from the roll 53A on the suction rotary drum 46. Since the suction rotary drum sucks air through a suction zone 47, the applied super-absorbent polymer 31 is coated onto the first sheet 41. Next, the hot-melt adhesive is coated from the hot-melt adhesive coating machine 43 onto the second sheet 42 from the roll 53B. The first sheet 41 that has been coated with the super-absorbent polymer 31 is then laminated with the second sheet 42 coated with the hot-melt adhesive by the first attachment roll 48, with the super-absorbent polymer 31 and hot-melt adhesive sandwiched between them.

Next, the laminated layered absorbent sheet is conveyed by the belt conveyor 49 provided with a suction zone 47, and the layered absorbent sheet is cut to prescribed sizes by the layered absorbent sheet cutter 50 and then conveyed by the belt conveyor 49 provided with a suction zone 47. Next, a hot-melt adhesive is coated by the hot-melt adhesive coating machine 43 onto the front sheet 35 from the roll 53C, a hot-melt adhesive is coated by the hot-melt adhesive coating machine 43 onto the back sheet 36 from the roll 53D, and the second attachment rolls 51 are used to bond the front sheet 35 and the back sheet 36 in a manner sandwiching the layered absorbent sheet 40. The product cutter 52 is then used to cut the connected absorbent article into discrete absorbent articles 34.

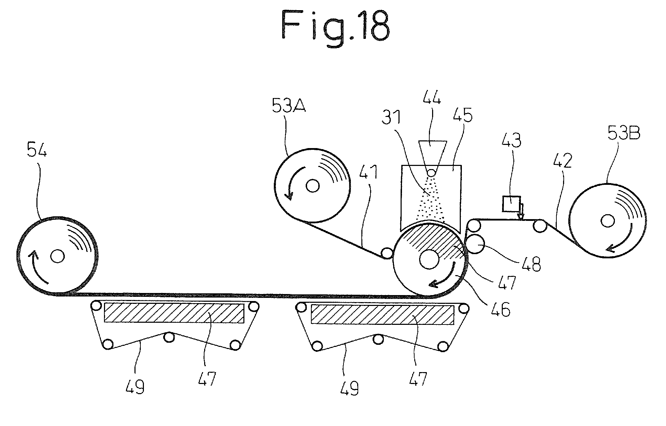

As shown in Fig. 18, a take-up roll 54 of the layered absorbent sheet can be obtained by taking up the laminated layered absorbent sheet onto a roll.

When the fiber of the bulky absorbent sheet in the absorbent article has a pulp body, the pulp is easily softened by absorption of moisture upon being worn, and then the expanded heat-expanding particles serve the role of a sponge cushion, thus providing a satisfactory feel during wear.

The absorbent article may be a panty liner, sanitary napkin, paper diaper, perspiration sheet, pet sheet, or absorbing sheet for drip of foods, such as meat or fish.

Examples

The invention will now be explained in greater detail using examples and comparative examples, with the understanding that the invention is in no way limited by the examples.

[Production Example 1]

- Production of bulky absorbent sheet 1 -

After (i) 70 parts by mass of unbeaten Northern bleached Kraft pulp and 15 parts by mass of 2.2 dtex x 5 mm PP/PE core-sheath composite fiber (NBF, product of Daiwabo Polytec Co., Ltd.) as fiber starting materials, (ii) 15 parts by mass of low-boiling-point solvent-encapsulating microcapsules (FUC-36 Matsumoto Microspheres, product of Matsumoto Yushi-Seiyaku Co., Ltd., particle size: 5-15 $\mu$m, initial expansion temperature: 75-85°C) as heat-expanding particles, and (iii) as auxiliary agents, 0.5 part by mass (as the active ingredient) of a cation-modified acrylic copolymer (FILEX RC-104 by Meisei Chemical Works, Ltd.) and 0.3 part by mass (as the active ingredient) of an acrylic copolymer (FILEX M by Meisei Chemical Works, Ltd.), as agents for anchoring the heat-expanding particles to the pulp, 0.5 part by mass (as the active ingredient) of a polyamide/epichlorhydrin resin (WS4024, wet strength agent by Seiko PMC Corp.) as a paper strength additive, and 0.4 part by mass (as the active ingredient) of a fatty acid amide (PROSOFT TQ218 by Hercules, Inc.) as a hydrophilic flexibilizer, were mixed in water, an aqueous dispersion was formed to obtain a 2 mass% paper-making starting material 1.

The paper-making starting material 1 was used to produce paper with a basis weight of 50 g/m$^2$ using a rectilinear handsheet machine (80 mesh) according to a common method, and the paper was dewatered by sandwiching between filter sheets to obtain a wet blended sheet with a water content of 60 mass%. The paper-making wire of the handsheet machine was the one shown in Fig. 9. The screened wet blended sheet was placed on a conveyor belt and transported at a speed of 5 m/min. During this time, water vapor obtained from a boiler (nozzle manifold internal temperature: 172-174°C, pressure: 0.82-0.85 MPa) was sprayed from a nozzle (hole diameter: 0.3 mm, hole pitch: 2 mm, single row arrangement) through a 90 mesh wire mesh, from the top side of the wet blended sheet, and suction was applied from the bottom of the conveyor belt, to cause expansion of the sheet. Next, the sheet was dried with a rotary dryer set to 120°C, without applying strong pressure thereto, to obtain a bulky absorbent sheet 1 with a basis weight of 50 g/m$^2$. The bulky absorbent sheet 1 had a concavoconvex pattern with depressed low-basis-weight regions in a circular island pattern interspersed in high-basis-weight region, and the degree of expansion of the heat-expanding particles was approximately the same in both regions. The high-basis-weight region had a basis weight of about 59.1 g/m$^2$, a thickness of about 2.3 mm and a density of about 0.026 g/cm$^3$, while the low-basis-weight regions had a basis weight of about 20 g/m$^2$, a thickness of about 0.8 mm and a density of about 0.025 g/cm$^3$. The area of the low-basis-weight regions was 28% of the total.

[Production Example 2]

- Production of bulky water-absorbing sheet 2 -

A bulky water-absorbing sheet 2 without a concavoconvex pattern, having a basis weight of 50 g/m$^2$, a thickness of 1.6 mm and a density of 0.031 g/cm$^3$, was obtained in the same manner as Production Example 1, except that a non-blocked wire was used.

[Production Example 3]

- Production of water-resistant tissue paper 1 -

Water-resistant tissue paper 1 (basis weight: 50 g/m$^2$) was produced in the same manner as Production Example 1, except for excluding the low-boiling-point solvent-encapsulating microcapsules (Matsumoto Microsphere FUC-36 by Matsumoto Yushi-Seiyaku Co., Ltd.) as the heat-expanding particles and the cation-modified acrylic copolymer (FILEX RC-104 by Meisei Chemical Works, Ltd.) and the acrylic copolymer (FILEX M by Meisei Chemical Works, Ltd.), as the agents for anchoring the heat-expanding particles to the pulp.

[Production Example 4]

- Production of dry pulp nonwoven fabric 1 -

A commercially available dry pulp nonwoven fabric (KINOCLOTH, product of Oji Kinocloth Co., Ltd., basis weight: 48 g/m$^2$, thickness: 1.05 mm, density: 0.046 g/cm$^3$) was prepared, for use as a dry pulp nonwoven fabric 1. The dry pulp nonwoven fabric 1 was a nonwoven fabric produced by spraying an acrylic resin emulsion binder onto a fibrous mat obtained by laminating dry macerated pulp fiber, and then drying it.

[Production Example 5]

- Production of layered absorbent sheet 1 -

As a super-absorbent polymer there was prepared a sodium polyacrylate-based absorber with a center particle size of 300-500 $\mu$m (PA-800 by San-Dia Polymers, Ltd.).
The bulky water-absorbing sheet 1 obtained in Production Example 1 was placed on the top side of the concavoconvex pattern, on a support net. The super-absorbent polymer was dispersed at 50 g/m$^2$ from the top side while suctioning air from the bottom side of the bulky water-absorbing sheet 1. Next, the water-resistant tissue paper 1 produced in Production Example 3 was integrally layered in a manner sandwiching the super-absorbent polymer, to obtain a layered absorbent sheet 1. A hot-melt pressure-sensitive adhesive was coated in a spiral pattern on the water-resistant tissue paper 1 before integration. The super-absorbent polymer was present mostly in the recesses of the bulky absorbent sheet 1, i.e. at the low-basis-weight regions, but a portion was also present in the heights of the bulky absorbent sheet 1, i.e. at the high-basis-weight regions.

[Production Example 6]

- Production of layered absorbent sheet 2 -

A layered absorbent sheet 2 was obtained in the same manner as Example 1, except for using the bulky water-absorbing sheet 2 produced in Production Example 2 instead of the water-resistant tissue paper 1.

[Comparative Production Example 1 and Comparative Production Example 2]

- Production of layered absorbent sheet 3 and layered absorbent sheet 4 -

Layered absorbent sheet 3 and layered absorbent sheet 4 were each produced in the same manner as Production Example 5, except that the bulky absorbent sheet 1 of the layered absorbent sheet 1 produced in Production Example 5 was changed to a water-resistant tissue paper 1 and a dry pulp nonwoven fabric 1, respectively.
By partially blocking the suction support net during production, the super-absorbent polymer particles were sucked and arranged in a concentrated manner onto the non-blocked sections, so that the super-absorbent polymer was arranged in approximately the same pattern as the bulky absorbent sheet 1.

[Examples 1 and 2, Comparative Examples 1 and 2]

The layered absorbent sheets 1 and 2 as Examples 1 and 2, and the layered absorbent sheets 3 and 4 as Comparative Examples 1 and 2, respectively, were subjected to the following tests. The results are shown in Table 1.

<Maximum absorption>

A 5 cm-square layered absorbent sheet placed on wire mesh was dipped in physiological saline for 10 minutes. After 10 minutes, it was removed and placed horizontally, measuring the mass after water droplets no longer ran off, and the absorption per 1 g of layered absorbent sheet was recorded as the maximum absorption.

<Absorption rate>

A 20 mL portion of physiological saline was dropped from a burette onto the center section of a 15 cm-square layered absorbent sheet, while adjusting the amount to avoid run-off from the layered absorbent sheet, and the time required for the total amount to be absorbed into the sheet was measured as the absorption rate (sec/20 mL) and recorded as the first absorption rate.
After 3 minutes, an additional 20 mL of physiological saline was dropped, and the absorption rate (sec/20 mL) was measured and recorded as the second absorption rate.

<Rewetting amount>

After 10 minutes had elapsed from measurement of the second absorption rate, 10 sheets of 15 cm-square filter paper (No.2, product of Advantech Toyo, Inc.) were stacked onto the layered absorbent sheet, a load of 35 g/cm$^2$ was applied for 1 minute from the top of the filter paper, and the amount of physiological saline absorbed by the filter paper was recorded as the rewetting amount.

<Width compression recoverability>

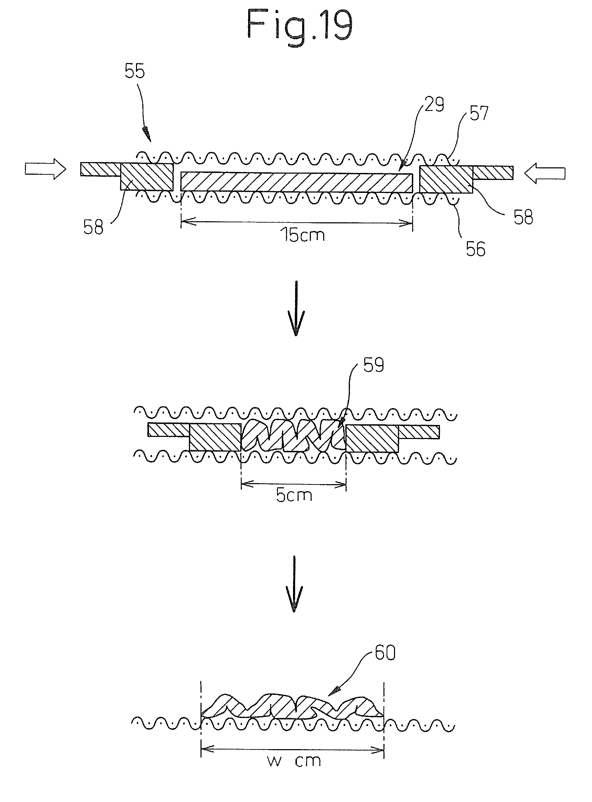

The width compression recoverability meter 55 shown in Fig. 19 was used. After measurement of the rewetting amount, the layered absorbent sheet 29 was mounted on a 10-mesh wire mesh 56 and covered with a similar 10-mesh wire mesh 57 at a height of 2 cm from the wire mesh 56, and then compressed with a pusher 58 from the 2 opposite directions (left and right in Fig. 19) to a layered absorbent sheet 29 width of 5 cm, thus forming a compressed layered absorbent sheet 59. The pusher 58 and wire mesh 57 were then removed, the width w (cm) of the recovered layered absorbent sheet 60 was measured, and the width compression recoverability (%) was calculated by the following formula.

```
Width compression recoverability (%) = {(w-5)÷(15-5)} ×
100
```

Table 1

| | Example 1 | Example 2 | Comp. Example 1 | Comp. Example 2 |
|---|---|---|---|---|
| Layered absorbent sheet No. | 1 | 2 | 3 | 4 |
| First sheet layer on absorbing side | Bulky absorbent sheet 1 | Bulky absorbent sheet 1 | Water-resistant tissue paper 1 | Dry pulp nonwoven fabric 1 |
| Super-absorbent polymer | PA-800 | PA-800 | PA-800 | PA-800 |
| Second sheet layer on non-absorbing side | Water-resistant tissue paper 1 | Bulky absorbent sheet 2 | Water-resistant tissue paper 1 | Water-resistant tissue paper 1 |
| Layered absorbent sheet thickness (mm) | 2.6 | 4.3 | 0.4 | 1.4 |
| Maximum absorption (g/g) | 15.2 | 15.5 | 15.8 | 16.7 |
| First absorption rate (sec/20 mL) | 14 | 10 | 33 | 14 |
| Second absorption rate (sec/20 mL) | 25 | 9 | 50 | 25 |
| Rewetting amount (g) | 11.4 | 11.2 | 11.6 | 16.0 |
| Width compression recoverability (%) | 31 | 63 | 0 | 7 |

As clearly seen from Table 1, the layered absorbent sheets 1 and 2 of the invention had excellent absorption rates and superior width compression recoverability compared to the prior art layered absorbent sheet 3. The layered absorbent sheets 1 and 2 of the invention also had superior width compression recoverability compared to the layered absorbent sheet 4 of Comparative Example 2. Particularly when both the first sheet and second sheet were bulky water-absorbing sheets, as in the layered absorbent sheet 2, not only the absorption properties but also the width compression recoverability was even more excellent.

References Signs List

1 Expanded heat-expanding particles
2 Fiber starting material
3 Cross-section of expanded heat-expanding particles
4 Paper machine
5 Paper-making part
6 Wet blended sheet
7 First conveyor belt
8 Second conveyor belt
9 Suction box
10 Jet nozzle
11 Screen
12 Dryer
13 Finished product take-up roll
14 Jet hole
15 Nozzle plate
16 Columnar jet

17 Spray slit
18 Curtain jet
19,19' Bulky absorbent sheets
20 Wavy line
21 Paper-making starting material solution
22 Paper-making cylinder
23 Screen drum
24 Paper-making wire
25 Blocked region
26 Non-blocked region
27 High-basis-weight region
28 Low-basis-weight region
29 Layered absorbent sheet
30 Bulky absorbent sheet
30' Bulky absorbent sheet with concavoconvex pattern
31 Super-absorbent polymer
32 Water-resistant tissue paper
33 Hot-melt adhesive
34 Absorbent article
35 Front sheet
36 Back sheet
37 Cohesive part
38 Detached portion
39 Fluff pulp layered mat
40 Layered absorbent sheet
41 First sheet
42 Second sheet
43 Hot-melt adhesive coating machine
44 Super-absorbent polymer applicator
45 Super-absorbent polymer application chamber
46 Suction rotary drum
47 Suction zone
48 First attachment roll
49 Belt conveyor
50 Layered absorbent sheet cutter
51 Second attachment roll
52 Product cutter
53A, 53B, 53C, 53D Rolls
54 Layered absorbent sheet take-up roll
55 Width compression recoverability meter
56,57 Wire meshes
58 Pusher
59 Compressed layered absorbent sheet
60 Recovered layered absorbent sheet

**Claims**

1. A layered absorbent sheet comprising a first sheet layer on the absorbing side, a second sheet layer on the non-absorbing side, and a super-absorbent polymer layer between the first sheet layer and second sheet layer:

   wherein the first sheet and/or second sheet are bulky absorbent sheets,
   the bulky absorbent sheets comprising heat-expanding particles that are dispersed in the bulky absorbent sheet and have been expanded by heat.

2. The layered absorbent sheet according to claim 1, wherein the bulky absorbent sheet is a bulky absorbent sheet with a density of 0.025-0.1 g/cm$^3$, obtained by a production method comprising the steps of:

forming a wet blended sheet comprising the heat-expanding particles dispersed in the fiber starting material from a paper-making starting material in which a fiber starting material comprising 30-100 mass% of natural pulp and 0-70 mass% of other fiber, and heat-expanding particles with a mean particle size of 5-30 μm, are dispersed in water at a proportion of 5-30 parts by mass of the heat-expanding particles per 100 parts by mass of the fiber starting material, and then

expanding at least some of the heat-expanding particles by heat to a 20-125 fold volume, to obtain a bulky absorbent sheet.

3. The layered absorbent sheet according to claim 2, wherein the heating is carried out by moist hot air or water vapor, and the expanding step is further followed by a step of drying the wet blended sheet.

4. The layered absorbent sheet according to any one of claims 1 to 3, wherein the bulky absorbent sheet has a concavoconvex pattern consisting of one or more high-basis-weight regions and one or more low-basis-weight regions.

5. The layered absorbent sheet according to claim 4, wherein the one or more low-basis-weight regions are interspersed within the high-basis-weight region.

6. The layered absorbent sheet according to any one of claims 1 to 5, wherein the bulky absorbent sheet has a concavoconvex pattern consisting of one or more low density regions with a high degree of expansion of the heat-expanding particles, and one or more high density regions with a low degree of expansion of the heat-expanding particles.

7. The layered absorbent sheet according to any one of claims 1 to 6, wherein both the first sheet and the second sheet are bulky absorbent sheets.

8. The layered absorbent sheet according to any one of claims 1 to 7, wherein the first sheet is the bulky absorbent sheet, and the second sheet is water-resistant tissue paper.

9. The layered absorbent sheet according to any one of claims 1 to 8, which further comprises a fluff pulp layered mat layer on the non-absorbing side of the second sheet.

# Fig.1

$100 \mu m$

# Fig.2

$66.6 \mu m$

# Fig.3

# Fig.4

# Fig.5

# Fig.6

CD DIRECTION

10

16

20

19

MD DIRECTION

# Fig.7

CD DIRECTION

CD DIRECTION

MD DIRECTION

10

16

20

19

10

16

20

# Fig.8

# Fig.9

# Fig.10

# Fig.11

# Fig.12

27   28

2.3mm

0.8mm

# Fig.13

## (a)

## (b)

## (c)

# Fig.14

32

33

30'

31

# Fig.15

A

34

35

36

A

# Fig.16

## (a)

## (b)

## (c)

Fig.17

# Fig.18

EP 2 484 321 A1

# Fig.19

55

29  57

58

15cm

58

56

59

5cm

60

w cm

<div align="center">

**INTERNATIONAL SEARCH REPORT**

</div>

| | International application No. |
|---|---|
| | PCT/JP2010/065083 |

**A. CLASSIFICATION OF SUBJECT MATTER**
*A61F13/49*(2006.01)i, *A61F13/53*(2006.01)i

According to International Patent Classification (IPC) or to both national classification and IPC

**B. FIELDS SEARCHED**

Minimum documentation searched (classification system followed by classification symbols)
A61F13/49, A61F13/53

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2010 |
| Kokai Jitsuyo Shinan Koho | 1971-2010 | Toroku Jitsuyo Shinan Koho | 1994-2010 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y | JP 2009-203586 A (Kao Corp.), 10 September 2009 (10.09.2009), entire text; all drawings (Family: none) | 1-9 |
| Y | JP 7-238451 A (Nippon Zeon Co., Ltd.), 12 September 1995 (12.09.1995), entire text; all drawings (Family: none) | 1-9 |
| Y | JP 2009-72420 A (Kao Corp.), 09 April 2009 (09.04.2009), paragraph [0094]; fig. 3 (Family: none) | 1-9 |

☒ Further documents are listed in the continuation of Box C.  ☐ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 17 November, 2010 (17.11.10) | 30 November, 2010 (30.11.10) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No.

PCT/JP2010/065083

C (Continuation). DOCUMENTS CONSIDERED TO BE RELEVANT

| Category* | Citation of document, with indication, where appropriate, of the relevant passages | Relevant to claim No. |
|---|---|---|
| Y | JP 7-163618 A (New Oji Paper Co., Ltd.), 27 June 1995 (27.06.1995), paragraph [0024] (Family: none) | 9 |

Form PCT/ISA/210 (continuation of second sheet) (July 2009)

## REFERENCES CITED IN THE DESCRIPTION

**Patent documents cited in the description**

- JP 8246395 A
- JP 2001171027 A
- JP 10118115 A